# EUROPEAN PATENT APPLICATION

(11) **EP 4 775 160 A2**
(43) Date of publication of application: **15.07.2026**
(21) Application number: 26150443.5
(22) Date of filing: 07.01.2026
(51) Int. Cl.: A61B 17/16

(54) **REAMING ASSEMBLIES AND ASSOCIATED METHODS FOR SURGICAL PROCEDURES**

(30) Priority: 09.01.2025 US 202563743266 P
(71) Applicant: Arthrex, Inc, Naples, FL 34108-1945 (US)
(72) Inventor: Khosla, Rudraksh, Naples, 34119 (US); Acker, Dean, Naples, 34109 (US); Wise, Mark, 46032 Carmel (IN)
(74) Representative: Lohr, Jöstingmeier & Partner Patent- und Rechtsanwälte mbB

(57) **Abstract**

This disclosure relates to surgical systems, devices and methods for planning and implementing surgical procedures. The systems and methods disclosed herein may be utilized to secure components of an assembly together. The assembly may have one or more components. The components may be rotated, translated and/or otherwise moved relative to each other to establish an assembled configuration. A component of the assembly may be captured between adjacent components of the assembly. A locking mechanism may secure the components to each other in the assembled configuration.

## Description

### CROSS-REFERENCE TO RELATED APPLICATIONS

This application claims the benefit of U.S. Provisional Application No. 63/743,266, filed January 9, 2025, which is incorporated herein by reference in its entirety.

### BACKGROUND

This disclosure relates to surgical systems and methods for surgical procedures, including techniques for securing components of an assembly together.

Severe bone loss may occur along the proximal humerus of a patient. A total shoulder arthroplasty may be performed to treat the bone loss. The surgeon may resect a portion of the proximal humerus. The surgeon may secure a prosthesis to the resected bone to restore functionality to the shoulder joint.

### SUMMARY

This disclosure relates to systems, devices and methods of performing a surgical procedure. An assembly may include one or more components. The components may be rotated, translated and/or otherwise moved relative to each other to establish an assembled configuration. A locking mechanism may secure the components to each other in the assembled configuration.

An assembly for a surgical procedure according to an implementation may include a drive shaft extending along a drive axis. The drive shaft may be adapted to engage a tool. A guide may be adapted for insertion into bone. A reamer may include a plurality of teeth adapted to remove bone in response to rotation about the drive axis. A twist-lock mechanism may releasably secure the reamer between the drive shaft and the guide in an assembled configuration.

An assembly for a surgical procedure according to another implementation may include a drive shaft. The drive shaft may be adapted to engage a tool. The assembly may include a cutting device. A guide may be adapted to engage bone to set an orientation of the cutting device. A twist-lock mechanism may be adapted to secure the drive shaft and the guide to each other in an assembled configuration.

An assembly for a surgical procedure according to another implementation may include a drive shaft extending along a drive axis. The assembly may include a device that may be adapted to engage bone. A twist-lock mechanism may be adapted to releasably secure the drive shaft and the device to each other in an assembled configuration. The twist-lock mechanism may include a plurality of bosses that may mate with a plurality of protrusions to limit axial movement between the drive shaft and the device relative to the drive axis. A plurality of locking tabs may be adapted to engage the respective bosses to block rotation between the drive shaft and the device when in a locked position, but may be adapted to permit relative rotation between the drive shaft and the device when in an unlocked position.

A method of assembly for a surgical device according to an implementation may include inserting a proximal end of a guide through a passageway in a cutting device. The guide may be adapted to set an orientation of the cutting device relative to bone. The method may include inserting the proximal end of the guide into a receptacle of a drive shaft. The method may include rotating the drive shaft and the guide relative to each other to establish an assembled configuration such that the cutting device may be releasably secured between the guide and the drive shaft.

The present disclosure may include any one or more of the individual features disclosed above and/or below alone or in any combination thereof.

The various features and advantages of this disclosure will become apparent to those skilled in the art from the following detailed description. The drawings that accompany the detailed description can be briefly described as follows.

### BRIEF DESCRIPTION OF THE DRAWINGS

Figure 1 discloses a perspective view of an assembly for a surgical procedure.
Figures 2-3 disclose an exploded view of the assembly of Figure 1.
Figure 4 discloses a drive shaft of the assembly of Figure 1 including a collar in phantom.
Figure 5 discloses a sectional view taken along an axis of the drive shaft of Figure 4.
Figure 6 discloses a cutting device and a guide of the assembly of Figure 1.
Figure 7 discloses an exploded view of the cutting device and the guide of Figure 6.
Figures 8-9 disclose a portion of the drive shaft with the collar in an unlocked position and a locked position.
Figures 10-11 disclose perspective views of collar in the locked and unlocked positions of Figures 8-9.
Figures 12A-12B disclose a sectional view of the assembly with the collar in the unlocked position.
Figures 13A-13B disclose the assembly of Figures 12A-12B with the collar in the locked position.
Figure 14 discloses the assembly taken along line 14-14 of Figure 13B.
Figure 15 discloses a perspective view of the collar in the locked position with the cutting device omitted.
Figure 16 discloses a method of assembly according to an implementation.

Like reference numbers and designations in the various drawings indicate like elements.

### DETAILED DESCRIPTION

This disclosure relates to systems, devices and methods of performing a surgical procedure. Implementations of a surgical assembly are disclosed. The assembly may include a locking mechanism adapted to secure (e.g., interlock) two or more components of the assembly together. In implementations, the assembly may be useful in removing tissue such as bone from the anatomy of a patient.

During reconstruction total shoulder arthroplasty, a proximal humeral bone may be resected in cases where severe proximal humeral bone loss may be present. After resecting or otherwise removing a portion of the humeral bone, bone resection (e.g., planing) may be performed to ensure the resected bone may be substantially orthogonal to an intramedullary (IM) canal of the humerus. A guide may be inserted into the IM canal to establish an orientation of the bone planar. Because the anatomy of the humerus may vary for the patient population, multiple planer diameters and/or IM planer guides may be provided to ensure that adequate bone may be removed (e.g., planed) and may remain orthogonal to the IM canal. The multiple planer diameters and/or IM planer guides may be provided by different surgical instruments, which may increase inventory requirements and/or surgical preparation time.

The disclosed surgical assemblies may include a drive shaft (e.g., driver), cutting device (e.g., reamer) and/or guide (e.g., pilot). The drive shaft may be secured to a tool, such as a drill. The drive shaft, cutting device and guide may be coupled together by a modular connection. A locking (e.g., twist-lock) mechanism may be adapted to interlock or otherwise secure the drive shaft, cutting device and guide to each other in the assembled configuration. The cutting device may be captured between the drive shaft and the guide. In implementations, the cutting device may be a reamer (e.g., bone planar). The reamer may be utilized to form one or more flat (e.g., planar) cuts in bone. The guide may be insertable in a recess in bone, such as the IM canal of a long bone such as the humerus. The guide may set an orientation of the assembly, including the cutting device.

Assembly may include one or more of the following steps. The cutting device may be slid over the guide. The cutting device may have a geometry which may allow it to key into the guide, which may allow the cutting device and guide to rotate together as a unit. The drive shaft may be slid over the guide to capture the cutting device between the drive shaft and the guide. The guide may include one or more bosses. The drive shaft may include one or more protrusions (e.g., lips), which may be slid along slots between the adjacent bosses of the guide. The drive shaft may include one or more slots between the adjacent protrusions. The drive shaft may carry a collar. Movement of the bosses along respective slots in the drive shaft may cause the bosses to engage the collar, which may cause compression of a spring member. The spring member may be adapted to bias the collar from an unlocked position towards a locked position. The drive shaft and the guide may be rotated relative to each other to cause engagement between the bosses and the protrusions of the drive shaft. The protrusions of the drive shaft may slide under the bosses of the guide. The drive shaft and the guide may be rotated relative to each other until the collar may move distally (e.g., descend) and may lock the drive shaft and guide relative to each other. The collar may slide between the bosses of the guide to lock the assembly. The components of the assembly may rotate together as a unit when in the locked position.

To dissemble the components of the assembly, the surgeon or clinical user may pull or otherwise actuate the collar. The collar may be moved proximally to disengage the collar from the bosses of the guide. The drive shaft and guide may be rotated relative to each other such that the bosses and the protrusions of the drive shaft may disengage. The shaft and guide may be moved apart (e.g., pulled) to release the drive shaft and the guide from each other.

An assembly for a surgical procedure according to an implementation may include a drive shaft extending along a drive axis. The drive shaft may be adapted to engage a tool. A guide may be adapted for insertion into bone. A reamer may include a plurality of teeth adapted to remove bone in response to rotation about the drive axis. A twist-lock mechanism may releasably secure the reamer between the drive shaft and the guide in an assembled configuration.

In any implementations, the plurality of teeth may be dimensioned to establish a planar cut in response to rotation of the reamer about the drive axis.

In any implementations, the twist-lock mechanism may include a plurality of bosses that may be adapted to mate with a plurality of protrusions.

In any implementations, the bosses may be distributed about an outer periphery of the guide. The protrusions may be distributed about an inner periphery of the drive shaft.

In any implementations, a collar may be moveable along the drive shaft between an unlocked position and a locked position. The collar may permit release of the twist-lock mechanism in the unlocked position, but may block release of the twist-lock mechanism in the locked position.

In any implementations, the collar may include a plurality of locking tabs that may be adapted to engage the plurality of respective bosses to block relative rotation between the drive shaft and the guide when in the locked position.

In any implementations, the drive shaft may include a shaft body and a plurality of slots that may extend from a distal end of the shaft body. The locking tabs may be circumferentially aligned with the respective slots relative to the drive axis.

In any implementations, the locking tabs may be moveable in an axial direction along the respective slots such that the locking tabs may be axially aligned with the bosses in the locked position to block movement of the bosses along the respective slots, but may be axially misaligned with the bosses in the unlocked position to permit movement of the bosses along the respective slots. The axial direction may be relative to the drive axis.

In any implementations, a spring member may be adapted to bias the collar toward the locking position.

In any implementations, the guide may include a key that may be adapted to mate with a keyway of the reamer in the assembled configuration.

In any implementations, the twist-lock mechanism may include a plurality of bosses along the guide that may mate with a plurality of protrusions along the drive shaft. The bosses may be established adjacent to the key.

An assembly for a surgical procedure according to another implementation may include a drive shaft. The drive shaft may be adapted to engage a tool. The assembly may include a cutting device. A guide may be adapted to engage bone to set an orientation of the cutting device. A twist-lock mechanism may be adapted to secure the drive shaft and the guide to each other in an assembled configuration.

In any implementations, the cutting device may be a reamer including a plurality of teeth that may be dimensioned to establish a planar cut.

In any implementations, a collar may be carried by the drive shaft. The collar may permit release of the twist-lock mechanism in an unlocked position, but may block release of the twist-lock mechanism in a locked position.

In any implementations, a proximal end of the guide may be received in an opening along a distal end of the drive shaft. The guide may include a key adjacent the proximal end of the guide. The key may be adapted to mate with a keyway of the cutting device such that the cutting device may be captured between the drive shaft and the guide in the assembled configuration.

An assembly for a surgical procedure according to another implementation may include a drive shaft extending along a drive axis. The assembly may include a device that may be adapted to engage bone. A twist-lock mechanism may be adapted to releasably secure the drive shaft and the device to each other in an assembled configuration. The twist-lock mechanism may include a plurality of bosses that may mate with a plurality of protrusions to limit axial movement between the drive shaft and the device relative to the drive axis. A plurality of locking tabs may be adapted to engage the respective bosses to block rotation between the drive shaft and the device when in a locked position, but may be adapted to permit relative rotation between the drive shaft and the device when in an unlocked position.

In any implementations, a reamer may include plurality of teeth dimensioned to establish a planar cut. The reamer may be captured between the drive shaft and the device in the assembled position.

In any implementations, a collar may be moveable along the drive shaft to establish the unlocked position and the locked position.

In any implementations, the drive shaft may include the protrusions. The device may include the bosses. The locking tabs may be interspersed with the protrusions.

In any implementations, the drive shaft may include a plurality of slots that may be interspersed with the protrusions. The locking tabs may be circumferentially aligned with the respective slots relative to the drive axis. The locking tabs may be axially aligned with the bosses relative to the drive axis in the locked position to block movement of the bosses along the respective slots, but may be axially misaligned with the bosses relative to the drive axis in the unlocked position to permit movement of the bosses along the respective slots.

A method of assembly for a surgical device according to an implementation may include inserting a proximal end of a guide through a passageway in a cutting device. The guide may be adapted to set an orientation of the cutting device relative to bone. The method may include inserting the proximal end of the guide into a receptacle of a drive shaft. The method may include rotating the drive shaft and the guide relative to each other to establish an assembled configuration such that the cutting device may be releasably secured between the guide and the drive shaft.

In any implementations, the step of inserting the proximal end of the guide through the passageway may occur such that a key of the guide may mate with a keyway of the cutting device to oppose relative rotation.

In any implementations, the step of rotating the drive shaft and the guide relative to each other may occur such that a plurality of protrusions along a periphery of the receptacle may engage respective bosses along a periphery the guide to limit axial movement of the guide relative to the drive shaft.

In any implementations, the method may include moving a collar along the drive shaft between an unlocked position and a locked position. The collar may permit relative rotation between the guide and the drive shaft in the unlocked position, but may block relative rotation between the guide and the drive shaft in the locked position.

In any implementations, the collar may include a plurality of locking tabs that may be engaged with the guide in the locked position, but may be disengaged with the guide in the unlocked position.

In any implementations, the method may include inserting a distal end of the guide into a recess in bone to set the orientation of the cutting device. The method may include rotating the drive shaft to cause the cutting device to remove a portion of the bone associated with the orientation.

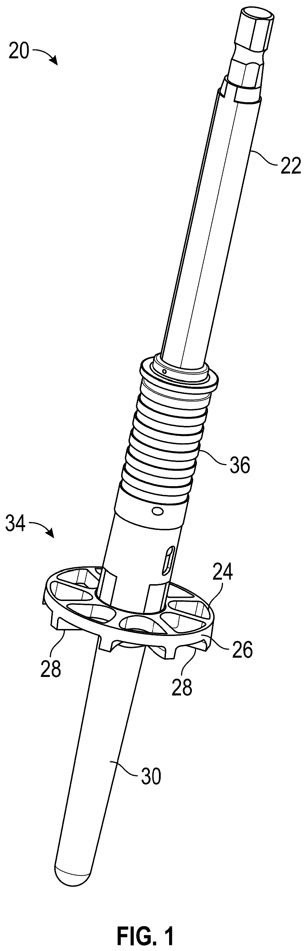

Figure 1 discloses a (e.g., reaming) assembly 20 for a surgical procedure according to an implementation. The assembly 20 may be useful for performing various surgical procedures, including orthopaedic procedures such as an arthroplasty for restoring functionality to various bones and joints (e.g., shoulder, ankle, hip, knee and elbow joints). In implementations, the assembly 20 may be a cutting instrument adapted to remove bone and/or other tissue.

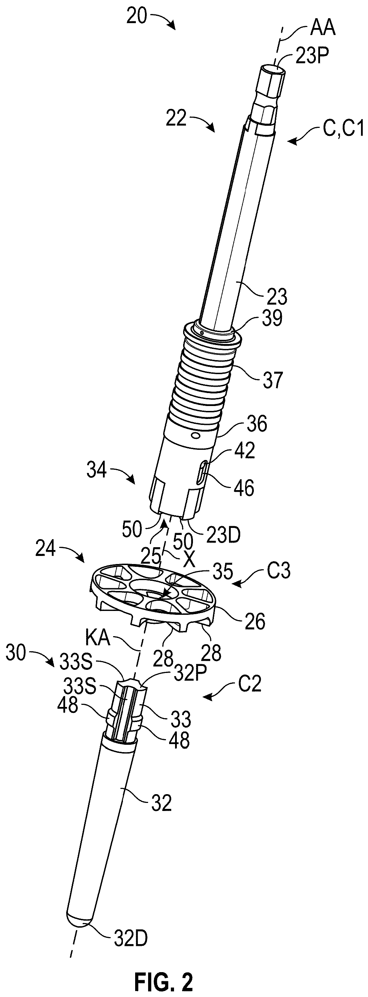

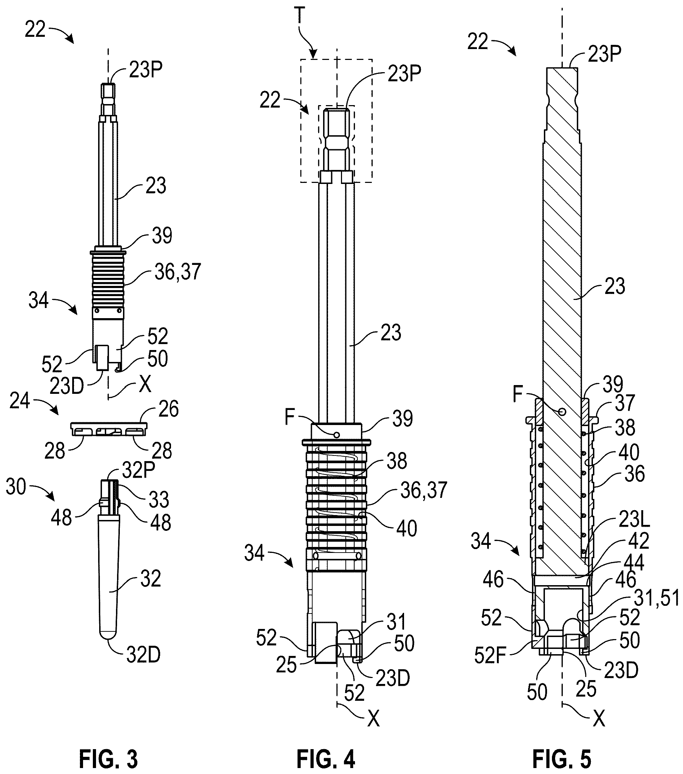

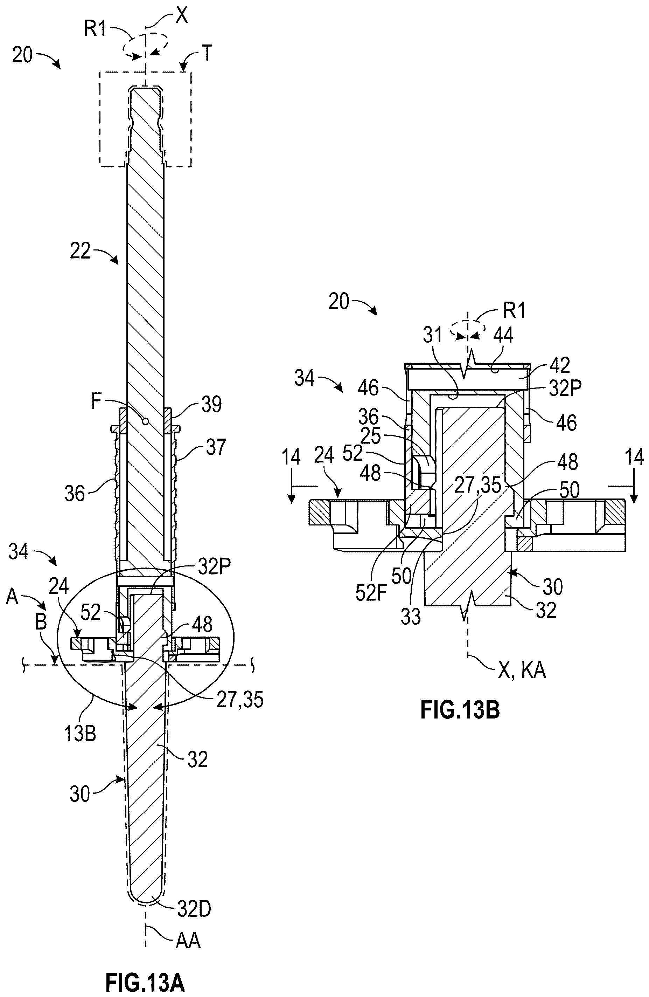

Referring to Figures 2-3, with continuing reference to Figure 1, the assembly 20 may include a drive shaft (e.g., driver) 22 and/or a cutting device (e.g., reamer or planar) 24. The drive shaft 22 may be adapted for engagement with a tool T, such as a drill (shown in dashed lines in Figures 4 and 13A). The drive shaft 22 may include an elongated shaft body 23. The shaft body 23 may extend along a drive axis X between a first (e.g., proximal) end 23P and a second (e.g., distal) end 23D. The shaft body 23 may include a recess (e.g., opening or receptacle) 31 (e.g., Figures 4-5). The receptacle 31 may be adapted to receive various devices. The receptacle 31 may be established along, and may extend inwardly from, the distal end 23D of the drive shaft 22.

The assembly 20 may include a guide (e.g., device or pilot) 30. The guide 30 may be adapted for positioning (e.g., orienting) the assembly 20 relative to the anatomy of a patient. In implementations, the guide 30 may be omitted. The cutting device 24 may be captured (e.g., trapped) between the drive shaft 22 and the guide 30 in an assembled position (e.g., Figures 1, 12A-12B and 13A-13B).

Various cutting devices 24 may be utilized, such as a drill bit or saw blade. In the implementation of Figures 2-3, the cutting device 24 may be a reamer (e.g., bone planer). The cutting device 24 may include a main body 26 and one or more cutting surfaces (e.g., teeth) 28. The main body 26 may have a disc-shaped geometry. The main body 26 may include a passageway 27. The passageway 27 may be dimensioned to receive a portion of the guide 30. The teeth 28 may be adapted to remove bone in response to rotation of the cutting device 24 about the drive axis X. The teeth 28 may be dimensioned to establish a planar cut in response to rotation of the cutting device 24 about the drive axis X. Cutting devices 24 and/or guides 30 of various shapes and/or sizes may be provided in a surgical kit to treat different anatomies. The surgeon or clinical user may select the appropriate cutting device 24 and/or guide 30 from the surgical kit for performing a surgical procedure for a patient.

The guide 30 may be adapted to engage bone to set an orientation of the cutting device 24. In the implementation of Figure 13B, the guide 30 may be adapted for insertion into bone B, such as within a recess R (e.g., drill hole or intramedullary canal). The guide 30 may have various geometries. In implementations, the guide 30 may have an elongated guide body 32 extending between a first (e.g., proximal) end 32P and a second (e.g., distal) end 32D. The guide body 32 may taper towards the distal end 32D. The distal end 32D may be rounded. The receptacle 31 of the drive shaft 22 may be dimensioned to receive the proximal end 32P and/or another portion of the guide body 30. The guide 30 may lack any cutting teeth. In other implementations, the guide 30 may include cutting teeth adapted to remove bone and/or other tissue.

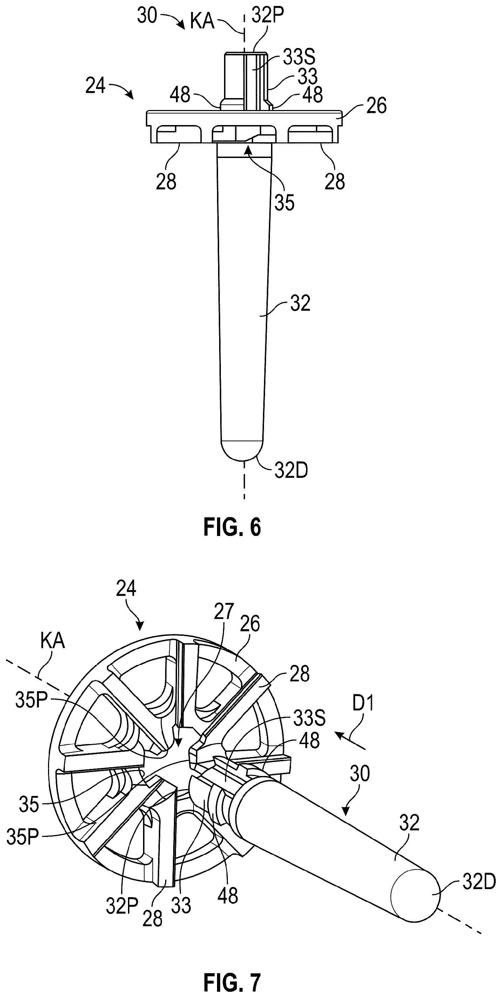

Various techniques may be utilized to limit relative rotation between the cutting device 24 and the guide 30. In the implementation of Figures 2 and 6-7, the assembly 20 may include a key 33 adapted to mate with a keyway 35 in the assembled configuration to secure the cutting device 24 and guide 30 to each other. In implementations, the guide 30 may include the key 33. The main body 26 of the cutting device 24 may include the keyway 35. The keyway 35 may be established along the passageway 27 of the cutting device 24. The key 33 may be established adjacent to the proximal end 32P of the guide 30. In the implementation of Figure 7, the key 33 may include one or more slots 33S. The slots 33S may be distributed about an axis KA of the guide 30. The slots 33S may be dimensioned to mate with respective protrusions 35P along the keyway 35.

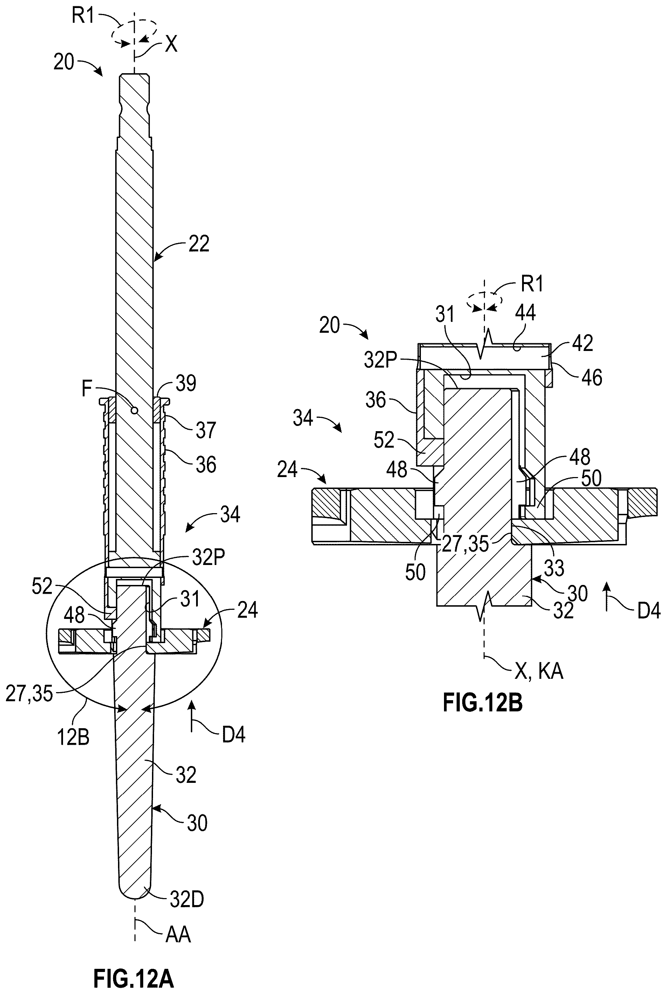

The guide 30 may be moveable in a first direction D1 (Figure 7) through the keyway 35 to establish engagement between the key 33 and keyway 35. In the implementation of Figures 12B and 13B, the proximal end 32P of the guide 30 may be receivable in the receptacle 31 of the drive shaft 22. The key 33 may be adapted to mate with the keyway 35 such that the cutting device 24 may be captured between the drive shaft 22 and the guide 30 in the assembled configuration.

Referring to Figures 2-5, with continuing reference to Figure 1, the assembly 20 may include a (e.g., twist-lock or locking) mechanism 34. The locking mechanism 34 may be adapted to (e.g., releasably) secure the drive shaft 22 and the guide 30 to each other in the assembled configuration. The locking mechanism 34 may be adapted to (e.g., releasably) secure the cutting device 24 between the drive shaft 22 and the guide 30 in the assembled configuration.

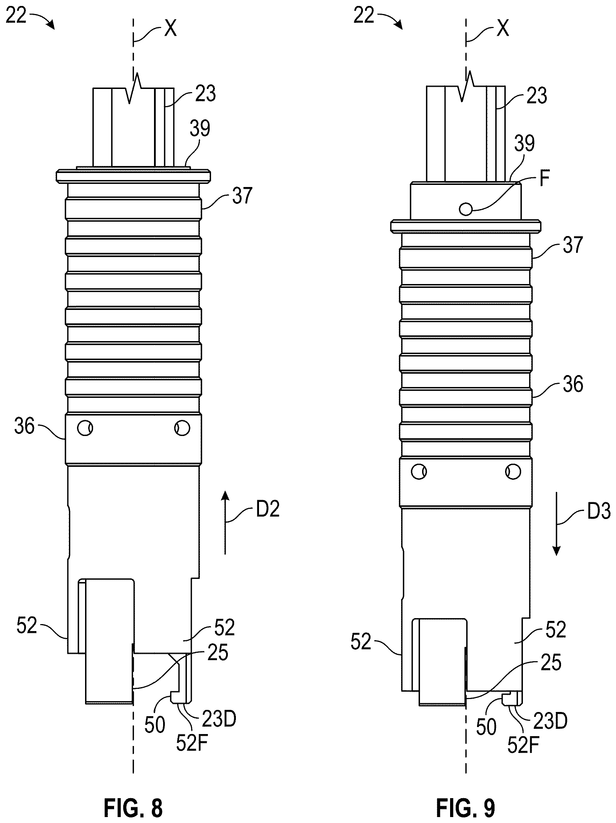

The assembly 20 may include a collar (e.g., lock) 36. The collar 36 may extend along the drive shaft 22. The collar 36 may include a (e.g., collar) passageway 40. The drive shaft 22 may extend through the collar passageway 40 (e.g., Figures 4-5). The collar 36 may be moveable along the drive shaft 22 between an unlocked position and a locked position. The collar 36 may be moveable in a second (e.g., axial) direction D2 along the drive shaft 22 to establish the unlocked position (e.g., Figure 8). The collar 36 may be moveable in a third (e.g., axial) direction D3 to establish the locked position (e.g., Figure 9). The second and third directions D2, D3 may be opposed to each other. The second and/or third directions D2, D3 may be substantially parallel to the drive axis X. The collar 36 may be adapted to permit release of the locking mechanism 34 in the unlocked position, but may be adapted to block release of the locking mechanism 34 in the locked position. The surgeon or clinical user may manipulate the collar 36 to move the collar 36 between the locked and unlocked positions.

Referring to Figures 4-5, with continuing reference to Figures 1-3, the assembly 20 may include a spring member 38. The spring member 38 may be adapted to bias the collar 36 toward the locking position. Various spring members 38 may be utilized, such as a coil or wave spring. The spring member 38 may be disposed about a periphery of the shaft body 23.

Various techniques may be utilized for securing the collar 36 to the drive shaft 22. The collar 36 may be carried by the drive shaft 22. The collar 36 may include a first portion (e.g., sleeve) 37 and/or a second portion (e.g., bushing) 39. The sleeve and bushing 37, 39 may be fixedly attached or otherwise secured to each other, such as with one or more fasteners F. In other implementations, the sleeve and bushing 37, 39 may be integrally formed. In the implementation of Figure 5, the spring member 38 may be captured between the sleeve 39 and a land portion 23L of the shaft body 23. A retention pin 42 may be received in a passage 44 of the drive shaft 22. The retention pin 42 may be received in one or more recesses (e.g., slots) 46 in the collar 36. The retention pin 42 may be dimensioned to engage opposite ends of the recess(es) 46 to limit axial movement of the collar 36 relative to the drive axis X of the drive shaft 22.

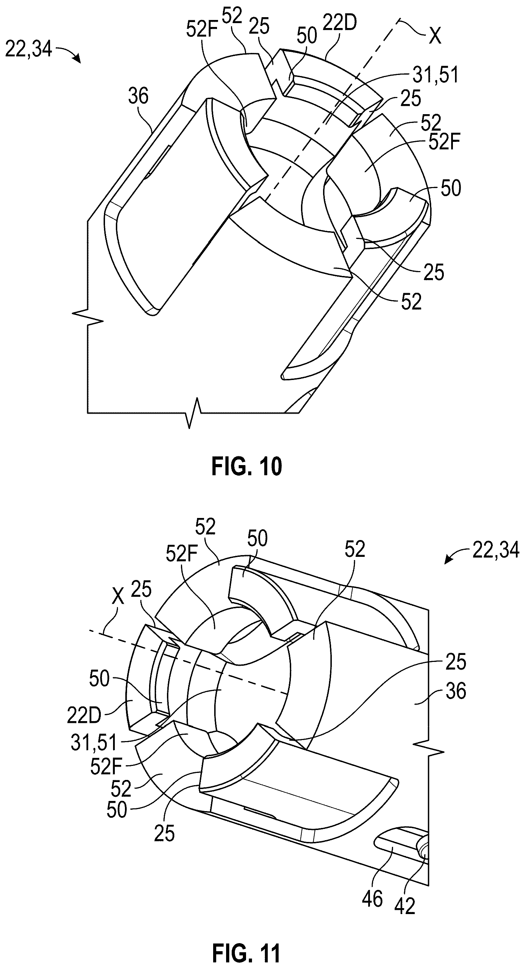

The locking mechanism 34 may include one or more engagement features to secure the drive shaft 22, cutting device 24 and/or guide 30 to each other. The locking mechanism 34 may include one or more bosses 48 and one or more protrusions 50. Each boss 48 may be adapted to mate with (e.g., engage) a respective one of the protrusions 50 (e.g., Figure 13B). The bosses 48 and protrusions 50 may cooperate to establish an interlock between the drive shaft 22, cutting device 24 and/or guide 30. In implementations, the guide 30 may include the bosses 48, and the drive shaft 22 may include the protrusions 50, or vice versa. The bosses 48 may be established along the guide 30. The bosses 48 may be distributed about an outer periphery of the guide 30. The bosses 48 may be established adjacent to the key 33. The protrusions 50 may be established along the drive shaft 22. The protrusions 50 may be distributed about an inner periphery 51 of the drive shaft 22. The inner periphery 51 may be established along the receptacle 31 (e.g., Figures 5 and 10-11). The locking mechanism 34 may include an equal number of the bosses 48 and protrusions 50. The bosses 48 may be adapted to mate with the respective protrusions 50 to limit axial movement between the drive shaft 22 and the guide (e.g., device) 30 relative to the drive axis X.

Referring to Figures 8-11, with continuing reference to Figures 1-5, the collar 36 may include one or more locking tabs 52. The locking tabs 52 may be distributed about the drive axis X. The locking tabs 52 may be interspersed with the protrusions 50. Each of the locking tabs 52 may include a respective locking flange 52F. The locking flanges 52F may be dimensioned to extend toward the drive axis DA. The locking flanges 52F may extend inwardly along the receptacle 31. The drive shaft 22 may include one or more slots 25 extending from the distal end 23D of the shaft body 23. The slots 25 may extend outwardly from the receptacle 31. The slots 25 may be interspersed with the protrusions 50. The locking tabs 52 may be circumferentially aligned with the respective slots 25 relative to the drive axis X. The locking tabs 52 may be translatable along the respective slots 25 between the locked and unlocked positions.

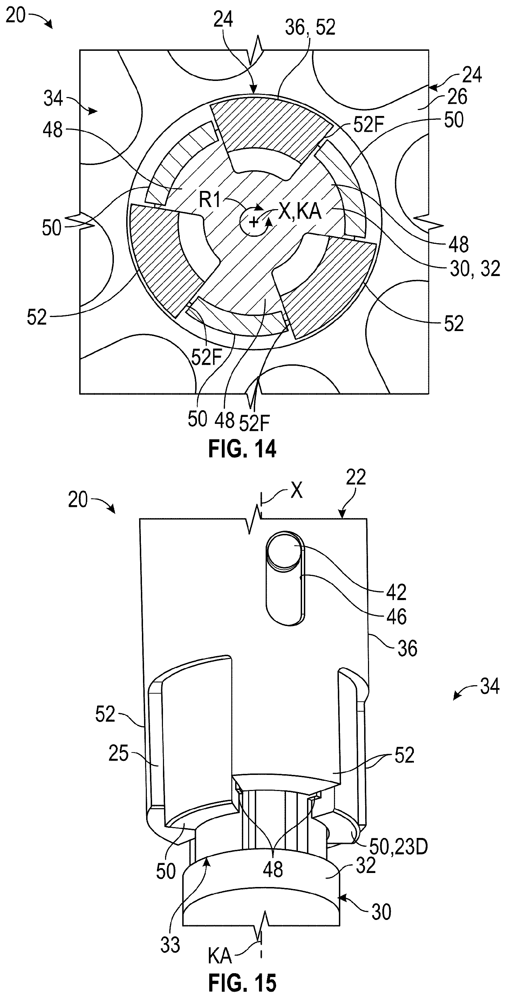

Figures 12A-12B, 13A-13B and 14 disclose aspects of the locking mechanism 34. Figures 12A-12B disclose the locking mechanism 34 in the unlocked position. Figures 13A-13B and 14 disclose the locking mechanism 34 in the locked position. Figure 15 discloses the locking mechanism 34 in the locked position with the cutting device 24 omitted. The locking tabs 52 may be adapted to engage the respective bosses 48 to block relative rotation between the drive shaft 22 and the guide 30 when in the locked position (e.g., Figures 13B and 14), but may be adapted to permit relative rotation between the drive shaft 22 and the guide 30 when in the unlocked position (e.g., Figure 12B). The relative rotation may be in a first rotational direction R1 relative to the drive axis X. The locking tabs 52 may be moveable in an (e.g., axial) direction (e.g., the second direction D2) along the respective slots 25 such that the locking tabs 52 may be axially aligned with the bosses 48 relative to the drive axis X in the locked position to block movement of the bosses 48 along the respective slots 48, but may be axially misaligned (e.g., offset) with the respective bosses 48 relative to the drive axis X in the unlocked position to permit movement of the bosses 48 along the respective slots 48. The locking tabs 52 may be axially aligned with the bosses 48 in the locked position to block relative rotation between the drive shaft 22 and the guide 30, but may be axially spaced apart from the bosses 48 in the unlocked position relative to the drive axis X to permit relative rotation between the drive shaft 22 and the guide 30. The drive axis X may be substantially collinear with an assembly axis AA of the assembly 20 in the assembled configuration (e.g., Figure 13A, see also Figures 2 and 12A).

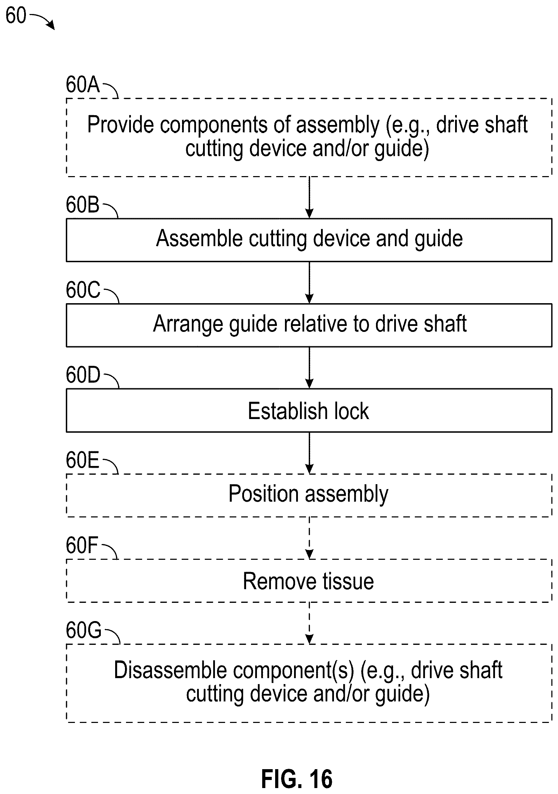

Figure 16 discloses a method of assembly for a surgical device in a flowchart 60 according to an implementation. The surgical device may be utilized to perform various surgical procedures. The surgical device may be utilized to perform various orthopaedic procedures such as an arthroplasty for restoring functionality to various bones and joints, such as shoulder, ankle, hip, knee and elbow joints. The method may be utilized with any of the surgical assemblies disclosed herein, such as the assembly 20. Fewer or additional steps than are recited below could be performed within the scope of this disclosure, and the recited order of steps is not intended to limit this disclosure. Reference is made to the assembly 20.

Referring to Figure 2, with continuing reference to Figure 16, one or more components C may be provided at block 60A. The components C may be provided in a surgical kit to the surgeon or clinical user. The components C may be arranged to establish the assembly 20. The components C may include any of the components disclosed herein, such as the drive shaft 22, cutting device 24 and/or guide 30. The components C may include a first component C1, a second component C2 and/or a third component C3. In the implementation of Figure 2, the first component C1 may include the drive shaft 22. The second component C2 may include the guide 30. The third component C3 may include the cutting device 24. In other implementations, the second component C2 may include the cutting device 24. In implementations, the components C2, C3 may be integrally formed. The second or third component C2, C3 may be omitted.

At block 60B, the cutting device 24 and guide 30 may be assembled together. The proximal end 32P of the guide 30 may be moved in the direction D1 (Figure 7) and may be inserted through the passageway 27 in the cutting device 24 (e.g., Figures 6 and 12A-12B). The guide 30 may be adapted to set an orientation of the cutting device 24 relative to bone.

At block 60C, the guide 30 may be arranged relative to the drive shaft 22 to establish an assembled configuration of the assembly 20. The lock mechanism 34 may be unlocked and/or locked in the assembled configuration. In the implementation of Figures 12A-12B, the proximal end 32P of the guide 30 may be moved in a fourth direction D4 and may be inserted into the receptacle 31 of the drive shaft 22. The proximal end 32P of the guide 30 may be translated or otherwise moved along the drive axis X and into abutment with a portion of the drive shaft 22 bounding the receptacle 31. The cutting device 24 and guide 30 may be moved together as a unit (e.g., when keyed together). Inserting the proximal end 32P of the guide 30 through the passageway 27 may occur such that the key 33 of the guide 30 may mate with the keyway 35 of the cutting device 24 to oppose relative rotation. The bosses 48 may be slid or otherwise moved along the respective slots 25 of the drive shaft 22. Movement of the bosses 48 along the slots 25 may cause the bosses 25 to engage the locking tabs 52 of the collar 36, which may cause compression of the spring member 38 (e.g., Figures 4-5). The guide 30 may extend distally from the cutting device 24 in the assembled configuration.

At block 60D, a lock may be established to secure the drive shaft 22, cutting device 24 and/or guide 30 relative to each other. In the implementation of Figures 12A-12B, the drive shaft 22 and guide 30 may be rotated relative to each other in the first rotational direction R1 to establish the assembled configuration such that the cutting device 24 may be releasably secured between the guide 30 and the drive shaft 22. The drive shaft 22, cutting device 24 and/or guide 30 may be interlocked when the lock assembly 34 is in the locked position.

Referring to Figures 13A-13B, with continued reference to Figures 12A-12B and 16, the drive shaft 22 and guide 30 may be rotated relative to each other such that the protrusion(s) 50 along the periphery of the receptacle 31 may engage the respective boss(es) 48 along the periphery the guide 30 to limit axial movement of the guide 30 relative to the drive shaft 22 and/or drive axis X.

The lock assembly 34 may be moved from the unlocked position to the locked position. The collar 36 may be moved along, or otherwise relative to, the drive shaft 22 between the unlocked position and the locked position. The spring member 38 (e.g., Figures 4-5) may bias the collar 36 from the unlocked position to the locked position. The surgeon or clinical user may manually move the collar 36 between the unlocked and locked positions. In implementations, the spring member 38 may be omitted. The collar 36 may permit relative rotation between the guide 30 and the drive shaft 22 in the unlocked position, but may block relative rotation between the guide 30 and the drive shaft 22 in the locked position. The locking tab(s) 52 of the collar 36 may be engaged with the bosses 48 of the guide 30 in the locked position (e.g., Figures 13B and 14), but may be disengaged with the bosses 48 of the guide 30 in the unlocked position (e.g., Figure 12B).

Referring to Figure 13A, with continuing reference to Figure 16, the assembly 20 may be positioned relative to the anatomy A of a patient at block 60E. Positioning the assembly 20 may include inserting the distal end 32D of the guide 30 into a recess R in bone B to set the orientation of the cutting device 24 and/or an assembly axis AA of the assembly 20 (see also Figure 12A). In implementations, the bone B may be a long bone such as a humerus. The recess R may be an intramedullary canal.

At block 60F, the assembly 20 may remove a portion of tissue such as bone B from the anatomy. The drive shaft 22 may be rotated or otherwise moved to cause the cutting device 24 to remove a portion of the bone B associated with the orientation.

Referring to Figure 2, with continuing reference to Figure 16, the component(s) may be disassembled at block 60G. The lock assembly 34 may be moved from the locked position to the unlocked position such that the locking tabs 52 may permit relative rotation between the drive shaft 22 and guide 30 (e.g., Figure 12B). The drive shaft 22 and guide 30 may be rotated in the first rotational direction R1 relative to each other such that the bosses 48 and protrusions 50 may disengage each other (e.g., Figure 12B). The guide 30 may be removed from the receptacle 31. The guide 30 may be removed from the cutting device 24.

The novel devices and methods of this disclosure provide improved assembly of components of a surgical instrument, which may be used to perform various cutting operations. The disclosed techniques may be utilized to secure components of an assembly together utilizing a modular connection (e.g., locking mechanism), which may reduce surgical preparation time. The components may be secured together in a manner that may improve accuracy in forming one or more (e.g., planar) cuts in the anatomy of a patient. The modular connection may improve reusability of components. The disclosed locking mechanism may allow for ease of use in assembly and disassembly and may withstand the torsional forces that may occur during bone reaming.

Although the different non-limiting embodiments are illustrated as having specific components or steps, the embodiments of this disclosure are not limited to those particular combinations. It is possible to use some of the components or features from any of the non-limiting embodiments in combination with features or components from any of the other non-limiting embodiments.

It should be understood that like reference numerals identify corresponding or similar elements throughout the several drawings. It should further be understood that although a particular component arrangement is disclosed and illustrated in these exemplary embodiments, other arrangements could also benefit from the teachings of this disclosure.

The foregoing description shall be interpreted as illustrative and not in any limiting sense. A worker of ordinary skill in the art would understand that certain modifications could come within the scope of this disclosure. For these reasons, the following claims should be studied to determine the true scope and content of this disclosure.

## Claims

1. An assembly for a surgical procedure comprising:
a drive shaft extending along a drive axis and adapted to engage a tool;
a guide adapted for insertion into bone;
a reamer including a plurality of teeth adapted to remove bone in response to rotation about the drive axis; and
a twist-lock mechanism that releasably secures the reamer between the drive shaft and the guide in an assembled configuration.

2. The assembly as recited in claim 1, wherein:
the plurality of teeth are dimensioned to establish a planar cut in response to rotation of the reamer about the drive axis.

3. The assembly as recited in claim 1 or 2, wherein the twist-lock mechanism includes a plurality of bosses adapted to mate with a plurality of protrusions, and optionally wherein:
• the bosses are distributed about an outer periphery of the guide; and
• the protrusions are distributed about an inner periphery of the drive shaft.

4. The assembly as recited in any preceding claim, further comprising:
a collar moveable along the drive shaft between an unlocked position and a locked position;
wherein the collar permits release of the twist-lock mechanism in the unlocked position, but blocks release of the twist-lock mechanism in the locked position.

5. The assembly as recited in claim 4, wherein:
the collar includes a plurality of locking tabs adapted to engage a/the plurality of respective bosses to block relative rotation between the drive shaft and the guide when in the locked position.

6. The assembly as recited in claim 5, wherein:
the drive shaft includes a shaft body and a plurality of slots extending from a distal end of the shaft body; and
the locking tabs are circumferentially aligned with the respective slots relative to the drive axis, and wherein:
the locking tabs may be moveable in an axial direction along the respective slots such that the locking tabs are axially aligned with the bosses in the locked position to block movement of the bosses along the respective slots, but are axially misaligned with the bosses in the unlocked position to permit movement of the bosses along the respective slots, the axial direction relative to the drive axis.

7. The assembly as recited in any of claims 4 to 6, further comprising:
a spring member adapted to bias the collar toward the locking position.

8. The assembly as recited in any preceding claim, wherein:
the guide includes a key adapted to mate with a keyway of the reamer in the assembled configuration, and
optionally wherein:
• the twist-lock mechanism includes a plurality of bosses along the guide that mate with a plurality of protrusions along the drive shaft; and
• the bosses are established adjacent to the key.

9. An assembly for a surgical procedure comprising:
a drive shaft adapted to engage a tool;
a cutting device;
a guide adapted to engage bone to set an orientation of the cutting device; and
a twist-lock mechanism adapted to secure the drive shaft and the guide to each other in an assembled configuration, and
wherein the cutting device may be a reamer including a plurality of teeth dimensioned to establish a planar cut, and
the assembly may further comprise:
a collar carried by the drive shaft; wherein the collar permits release of the twist-lock mechanism in an unlocked position, but blocks release of the twist-lock mechanism in a locked position, and
optionally wherein:
• a proximal end of the guide is received in an opening along a distal end of the drive shaft; and
• the guide includes a key adjacent the proximal end of the guide, and the key is adapted to mate with a keyway of the cutting device such that the cutting device is captured between the drive shaft and the guide in the assembled configuration.

10. An assembly for a surgical procedure comprising:
a drive shaft extending along a drive axis;
a device adapted to engage bone; and
a twist-lock mechanism adapted to releasably secure the drive shaft and the device to each other in an assembled configuration, the twist-lock mechanism comprising:
a plurality of bosses that mate with a plurality of protrusions to limit axial movement between the drive shaft and the device relative to the drive axis; and
a plurality of locking tabs adapted to engage the respective bosses to block rotation between the drive shaft and the device when in a locked position, but adapted to permit relative rotation between the drive shaft and the device when in an unlocked position;
and optionally comprising:
• a reamer including a plurality of teeth dimensioned to establish a planar cut;
• wherein the reamer is captured between the drive shaft and the device in the assembled position.

11. The assembly as recited in claim 10, further comprising:
a collar moveable along the drive shaft to establish the unlocked position and the locked position.

12. The assembly as recited in any of claims 10 to 11, wherein:
the drive shaft includes the protrusions;
the device includes the bosses; and
the locking tabs are interspersed with the protrusions, and
optionally wherein:
• the drive shaft includes a plurality of slots interspersed with the protrusions;
• the locking tabs are circumferentially aligned with the respective slots relative to the drive axis; and
• the locking tabs are axially aligned with the bosses relative to the drive axis in the locked position to block movement of the bosses along the respective slots, but are axially misaligned with the bosses relative to the drive axis in the unlocked position to permit movement of the bosses along the respective slots.

13. A method of assembly for a surgical device comprising:
inserting a proximal end of a guide through a passageway in a cutting device, the guide adapted to set an orientation of the cutting device relative to bone;
inserting the proximal end of the guide into a receptacle of a drive shaft; and
rotating the drive shaft and the guide relative to each other to establish an assembled configuration such that the cutting device is releasably secured between the guide and the drive shaft, and
wherein:
the step of inserting the proximal end of the guide through the passageway may occur such that a key of the guide mates with a keyway of the cutting device to oppose relative rotation, and
the step of rotating the drive shaft and the guide relative to each other may occur such that a plurality of protrusions along a periphery of the receptacle engage respective bosses along a periphery the guide to limit axial movement of the guide relative to the drive shaft.

14. The method as recited in claim 13, further comprising:
moving a collar along the drive shaft between an unlocked position and a locked position;
wherein the collar permits relative rotation between the guide and the drive shaft in the unlocked position, but blocks relative rotation between the guide and the drive shaft in the locked position, and
wherein the collar may include a plurality of locking tabs that are engaged with the guide in the locked position, but are disengaged with the guide in the unlocked position.

15. The method as recited in any of claims 13 to 14, further comprising:
inserting a distal end of the guide into a recess in bone to set the orientation of the cutting device; and
rotating the drive shaft to cause the cutting device to remove a portion of the bone associated with the orientation.
